Europäisches Patentamt

European Patent Office

Office européen des brevets

(19)

(11) Publication number: **0 119 373**
**B1**

(12) # EUROPEAN PATENT SPECIFICATION

(45) Date of publication of patent specification: **19.08.87**

(51) Int. Cl.⁴: **A 61 M 5/14, A 61 J 15/00**

(21) Application number: **83850075.9**

(22) Date of filing: **22.03.83**

(54) **Adapter for drop unit.**

(43) Date of publication of application:
**26.09.84 Bulletin 84/39**

(45) Publication of the grant of the patent:
**19.08.87 Bulletin 87/34**

(84) Designated Contracting States:
**AT BE CH DE FR GB IT LI LU NL**

(56) References cited:
**EP-A-0 067 737**
**DE-A-1 766 290**
**DE-A-2 409 339**
**DE-U-7 837 789**
**GB-A-2 018 720**
**GB-A-2 079 157**
**US-A-3 659 629**
**US-A-3 831 814**
**US-A-3 990 444**
**US-A-4 324 238**

(73) Proprietor: **SJÖNELL, Göran**
**Askrikevägen 11**
**S-181 46 Lidingö (SE)**

(72) Inventor: **SJÖNELL, Göran**
**Askrikevägen 11**
**S-181 46 Lidingö (SE)**

(74) Representative: **Onn, Thorsten et al**
**AB STOCKHOLMS PATENTBYRA Box 3129**
**S-103 62 Stockholm (SE)**

Courier Press, Leamington Spa, England.

## Description

This invention relates to a device for emptying a drip unit through a chamber, application hose, clip and probe for enteral nutrition. The artificial supply, for example, of nutrients usually is carried out by means of drip, enteral nutrition or parenteral nutrition. The package, usually a flask, containing the nutrient is suspended upside down, and the nutrient is supplied via a drop unit either intravenously or via a probe directly to the stomach. When the nutrient is supplied from a flask, the visual observation of the filling degree of the flask does not involve problems.

For economic as well as hygienic reasons, disposal packages holding the nutrient are now being used to an increasing extent, which packages are intended for direct use at the application occasion. In view of varying storage times and of the necessity of protecting the contents in the package against exposure to light for a longer period, the disposable package must be capable of preventing light from contacting the contents, which means that the packing material must bar the light and, consequently, is non-transparent. To ensure that the package is not emptied without the medical staff knowing about it, it is a fact based on experience that such disposal packages are exchanged with an ample time margin. This implies waste, which in view of the high costs of the packages and their contents involves for the health service at large very great. economic losses.

The conventional can packages, therefore, have not come into use to a large extent, however through EP—A—0 067 737 there is known an adapter rendering it possible to use can packages provided with sealed openings, which have to be opened before the application of the adapter.

The present invention as it is defined in the characterizing clauses of the claims, has the object to render it possible to use disposable packages in the form of closed cans *inter alia* for parenteral and enteral nutrition. The invention renders it possible then to provide completely unbroken packages, lacking any type of closure which has to be broken up and torn off, being a safe, simple and low price solution e.g. in the field of medical treatment.

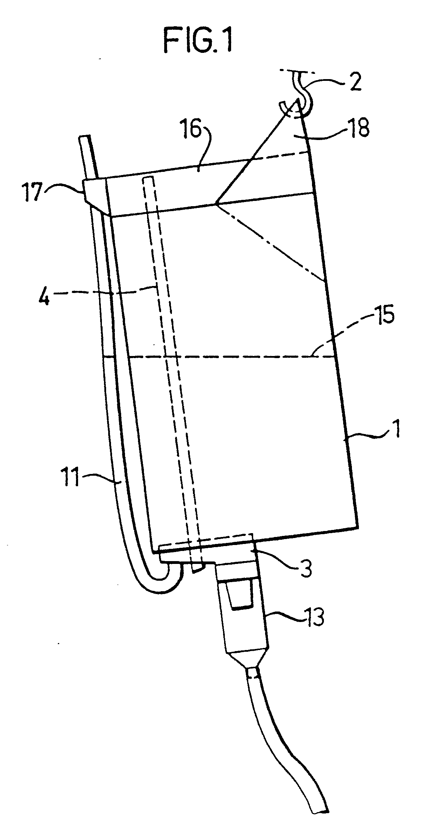

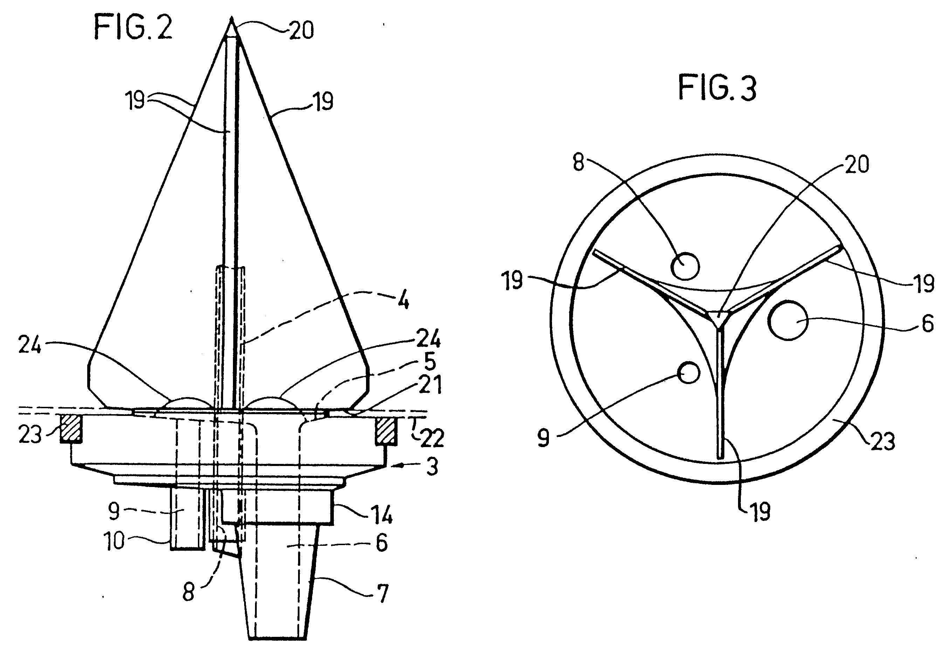

The invention is described in greater detail in the following, with reference to the accompanying drawings, in which Fig. 1 shows the invention applied to a disposal package in the form of a can, Fig. 2 is a side view of the plug-like portion of the invention, and Fig. 3 is a view from above of the portion according to Fig. 2.

The disposal package in the form of a sheet metal can is designated by 1. This package is provided in known manner at one end surface of the can with a closure in the form of a notch defining the opening. The notch can be broken up and torn off by means of a grab ring. The can 1 is shown suspended in a suitable way, for example by means of a hook 2. In the opening a main member or adapter member 3 in the form of a plug is attached which will be described with reference to Figs. 2 and 3. From said main member 3 an aeration tube 4 extends all the way to the vicinity of the can bottom and connects the can interior to the atmosphere.

Fig. 2 is a side view of the member 3. From a recess 5 the member facing to the can interior, a first through hole 6 extends and terminates in a spout 7. A second through hole 8 is provided to receive the aforesaid aeration tube 4. The member 3 further is provided with a third through hole 9, which preferably opens into a flange 10 formed in the member. To said flange a tube or hose 11 is to be connected, as will be described below.

The member 3 thus, comprises the three through holes 6, 8 and 9, the spout 7 and flange 10. The member also shows the shoulder 14 for the chamber 13 in the drop unit. The adapter is provided with a cutting means known per se in the form of a triangular pyramid body, the edges 19 of which form walls terminating in a tip 20. The body properly can be manufactured of hard plastic, while the tip 20 is of metal. The base of the edges or cutting edges 19 extends with an inclined surface 21 slightly outside the connection of this annular adapter member 3 to the cutting body. The upper boundary surface of the adapter member 3 is located at a short distance from the base edge of the cutting edges 19. This distance corresponds in principle to the sheet metal thickness of the can in the place where the adapter is to be attached. The can metal sheet is shown in Fig. 2 and designated by 22. The adapter member 3 further is provided with a gasket 23. For rendering the recess 5 of the member 3 capable to act in the way intended, the disc-shaped edges are provided in the base portion with recesses 24 and thereby ensure supply flow to the hole 6.

The chamber 13 (Fig. 1) included in conventional manner in the drip unit is intended to be attached with press fit on a shoulder 14 in the member, which shoulder coaxially surrounds said first hole 6. The drip unit further according to a known pattern comprises application hose, clip and probe.

The transparent tube or hose 11 is intended to be held extending along the outer surface of the can, whereby the liquid level 15 in question in the package easily can be observed in the tube 11. For correctly supporting the tube 11, the package is provided with a socket 16 about the can bottom, which socket includes a slit or aperture 17 holding the tube in the position shown in Fig. 1.

The socket 16 further is provided with a flap 18, which normally is folded down to abut the side of the can, as indicated by dash dotted line. When the can is to be suspended on the hook 2, the flap is drawn in the longitudinal direction of the can past the bottom, as shown in Fig. 1. The flap is provided with a hole for the hook 2.

The can preferably can be delivered with the socket 16 in place, and the member 3 with the drop unit as an accessory set. When the package is to be used, the adapter 3 is used for bringing about a hole in the can material, in that the cutting edge of the adapter with the edges 19 is pressed through the can material until the adapter member 3 with the gasket 23 abuts the same. The adapter thereafter is slightly turned so that the can material engages beneath the projecting edges 21 of the cutting edges. The adapter now is sealingly attached to the can bottom. The aeration tube 4 is inserted through the opening 8, and the adapter is ready for use. It may be suitable, depending on the appearance and size of the adapter member 3, to provide the member with some kind of handle or hold (not shown) for applying the adapter to and turning it through the can material. The drop unit with chamber 13 is already applied on the member 3, or it may at this step be applied thereon. The can now is turned to its position of use, and the flap is drawn up to the position shown in Fig. 1. The can is then ready to be suspended and used.

The device according to the invention, of course, can be designed in different ways within the scope of the invention. The position of the holes in the main member, for example, can be varied relative to each other, and also the means for applying the chamber on the member. The tube 11 may be a hose or a rigid tube bent in a suitable way. The socket 16, of course, can be formed with a plane flange or with radially directed loops for applying the tube 11 and for suspension on the hook 2.

## Claims

1. A device for emptying a drip unit through a chamber (13), application hose, clip and probe and intended for use with disposable packages, which device comprises a main member (3) intended to be sealingly attached in an opening in the package (1), which during use occupies an inverted position, which main member (3) is provided with a first through hole (6) for emptying the contents of the package, characterized in that the main member is provided with a second through hole (8) provided with an aeration tube (4) extending to the bottom of the package, a third through hole (9) provided with a transparent tube (11) extending outside the package at least to the same level as the package bottom, a penetrating means (19, 20) of pyramid shape to penetrate the package material, of which penetrating means the corners located at the base are provided with notches (21) for receiving between them and the member the package wall and thereby locking the member to the package when the main member and therewith the penetrating means are turned and securing means (14) for the chamber (13), in combination with holding means (16) provided at the package (1) for holding the transparent tube (11).

2. A device as defined in claim 1, characterized in that the securing means consists of a shoulder (14) concentrically surrounding the first hole (6) and intended to receive the free edge of the chamber (13).

3. A device as defined in any one of the preceding claims, characterized in that the holding means has the shape of a socket (16) surrounding the bottom of the package (1), which socket is provided in the edge with an aperture (17) or slit intended to receive the transparent tube (11).

4. A device as defined in claim 3, characterized in that the socket (16) on the substantially diametrically opposed side of the aperture (17) or slit is provided with means (18) for suspending the package.

5. A device as defined in claim 4, characterized in that said suspending means consists of a flap (18) of the socket (16), which flap is located along the side of the package and provided with suspension hole, and for suspending the package is folded out past the bottom side of the package.

## Patentansprüche

1. Vorrichtung zum Ablass einer Tropfeneinheit durch eine Kammer (13), Anschlusschlauch, Klemme und Sonde und zur Anwendung mit Wegwerfverpackungen, welche Vorrichtung einen Hauptkörper (3) umfasst, der in einer Öffnung der Verpackung (1) abdichtend angebracht werden soll, die in Gebrauch eine umgekehrte Lage einnimmt, wobei der Hauptkörper (3) mit einem ersten durchgehenden Loch (6) zur Leerung des Inhalts der Verpackung versehen ist, dadurch gekennzeichnet, dass der Hauptkörper mit folgendem versehen ist: Ein zweites durchgehendes Loch (8) mit einem sich zum Boden der Verpackung erstreckenden Entlüftungsrohr (4), ein drittes durchgehendes Loch (9), das mit einem durchsichtigen, sich ausserhalb der Verpackung mindestens bis zu demselben Niveau wie der Verpackungsboden erstreckenden Rohr (11) versehen ist, ein pyramidenförmiges Penetriermittel (19, 20) zum Penetrieren vom Verpackungsmaterial, dessen an der Basis gelegene Ecken mit Einschnitten (21) versehen sind, um die Verpackungswand zwischen ihnen und dem Mittel aufzunehmen und dabei das Mittel an der Verpackung zu verschliessen, wenn der Hauptkörper und damit das Penetriermittel gewandt werden, sowie Sicherungsmittel (1) für die Kammer (13) zusammen mit Haltemitteln (16), die an der Verpackung (1) zum Festhalten des durchsichtigen Rohres (11) vorgesehen sind.

2. Vorrichtung nach Anspruch 1, dadurch gekennzeichnet, dass das Sicherungsmittel aus einem Kragen (14) besteht, der das erste Loch (6) konzentrisch umgibt und dazu bestimmt ist, die freie Kante der Kammer (13) aufzunehmen.

3. Vorrichtung nach einem der vorhergehenden Ansprüche, dadurch gekennzeichnet, dass das Sicherungsmittel die Form einer Muffe (16) hat, die den Boden der Verpackung (1) umgibt, welche Muffe an der Kante mit einer Öffnung (17) oder einem Schlitz versehen ist, der das durchsichtige Rohr (11) aufnehmen soll.

4. Vorrichtung nach Anspruch 3, dadurch gekennzeichnet, dass die Muffe (16) an der diame-

trisch entgegengesetzten Seite der Öffnung (17) oder des Schlitzes mit Mittel (18) zum Aufhängen der Verpackung versehen ist.

5. Vorrichtung nach Anspruch 4, dadurch gekennzeichnet, dass das erwähnte Aufhängemittel aus einer Lasche (18) der Muffe (16) besteht, welche Lasche längs der Seite der Verpackung placiert, mit einem Aufhängeloch versehen und zum Aufhängen der Verpackung an der Bodenseite der Verpackung vorbei ausgefaltet ist.

**Revendications**

1. Dispositif servant à vider un goutte-à-goutte par une chambre (13), tuyau d'application, pince et sonde, destiné pour être utilisé avec des emballages perdus et comprenant un corps principal (3) à fixer de manière étanche dans une ouverture pratiquée dans l'emballage (1) occupant en utilisation une position renversée, ledit corps principal (3) étant pourvu d'un premier trou débouchant (6) servant à vider le contenu de l'emballage, caractérisé en ce que le corps principal est pourvu d'un second trou débouchant (8) recevant un tube de ventilation (4) s'étendant jusqu'au fond de l'emballage, d'un troisième trou débouchant (9) recevant un tube transparent (11) s'étendant à l'extérieur de l'emballage au moins jusqu'au même niveau que celui du fond de l'emballage, d'un organe pénétrant (19, 20) de forme pyramidale servant à pénétrer la matière de l'emballage et dont les angles situés à la base compor- tente des entailles (21) permettant à la paroi de l'emballage de s'insérer entre celles-ci et le corps, verrouillant ainsi le corps par rapport à l'emballage lorsque le corps principal et l'organe pénétrant qu'il comporte sont tournés, et d'un organe (14) de retenue de la chambre (13), en combinaison avec un support (16) prévu sur l'emballage (1) pour maintenir en place le tube transparent (11).

2. Dispositif selon la revendication 1, caractérisé en ce que l'organe de retenue est constitué par un épaulement (14) entourant concentriquement le premier trou (6) et destiné à recevoir le bord libre de la chambre (13).

3. Dispositif selon l'une des revendications précédentes, caractérisé en ce que le support présente la forme d'un manchon (16) entourant le fond de l'emballage (1) et dont le rebord comporte une ouverture (17) ou fente destinée à recevoir le tube transparent (11).

4. Dispositif selon la revendication 3, caractérisé en ce que le manchon (16) comporte, de son côté sensiblement diamétralement opposé à celui de l'ouverture (17) ou de la fente, un organe (18) permettant de suspendre l'emballage.

5. Dispositif selon la revendication 4, caractérisé en ce que ledit organe de suspension est constitué par un volet (18) faisant partie du manchon (16), ledit volet étant situé le long du côté de l'emballage et percé d'un trou de suspension permettant de suspendre l'emballage après dépliage du volet au-delà du fond de l'emballage.

# FIG.1

FIG.2

20

19        19

24                24
                  5
                  21
23                22
                  3
9                 14
10                6

8                 7

FIG.3

8         20

19              19

9               6

                23

19

0 119 373